# EUROPÄISCHE PATENTSCHRIFT

(11) **EP 1 702 640 B1**
(45) Veröffentlichungstag und Bekanntmachung des Hinweises auf die Patenterteilung: **25.06.2008**
(21) Anmeldenummer: 05005724.9
(22) Anmeldetag: 16.03.2005
(51) Int. Cl.: A61M 25/00, A61M 27/00

(54) **Intracranial-Katheter**
Intracranial catheter
Cathéter intracranien

(43) Veröffentlichungstag der Anmeldung: 20.09.2006
(73) Patentinhaber: BrainLAB AG, 85551 Kirchheim/Heimstetten (DE)
(72) Erfinder: Hartlep, Andreas, Dr., 83629 Naring (DE); Pedain, Christoph, Dr., 81667 München (DE)
(74) Vertreter: Gassenhuber, Andreas

(56) Entgegenhaltungen:
- US-A- 5 423 774
- US-A1- 2002 087 143
- US-A1- 2003 135 200

## Beschreibung

Die vorliegende Erfindung bezieht sich auf einen Intracranial-Katheter, sowie auf ein Verfahren zur Bestimmung einer Außenform oder Länge eines solchen Intracranial-Katheters.

Es sind verschiedene Katheter bekannt, welche sich hinsichtlich ihrer Form und Größe unterscheiden, also zum Beispiel verschiedene Durchmesser und Längen aufweisen und eine Öffnung zur Abgabe einer Flüssigkeit an einem distalen Katheterende oder entlang des Katheters haben.

Für die direkte Behandlung eines erkrankten Körperbereiches, wie zum Beispiel für die Behandlung von Gehirntumoren oder neurodegenerativen Erkrankungen, ist es vorteilhaft, wenn Medikamente oder therapeutische Mittel direkt dem erkrankten Bereich im Körper zugeführt werden können. Um ein Medikament einem erkrankten Gewebe zuzuführen, wird ein Katheter in den Körper eingebracht, so dass eine Katheteröffnung in oder in der Nähe des erkrankten oder zu behandelnden Gewebes oder Bereiches liegt.

Jedoch ist es mit bisher zu diesem Zweck verwendeten pediatrischen Ventrikelkathetern nicht möglich, sicher und zuverlässig eine bestimmte Menge einer zu verabreichenden Substanz in einem gewünschten Bereich eines Körpers abzugeben, da eine Flüssigkeit, welche aus einer Katheteröffnung austritt, zunächst größtenteils entlang der Oberfläche des Kathethers fließt, welcher die Gewebematrix durchstochen hat, und so entlang der Kathetertrajektorie von dem zu behandelnden Bereich wegfließt. Dieses Rückfluss-Phänomen wird "backflow" genannt, wobei die Rückflusslänge L proportional zur fünften Wurzel aus Q³ x r⁴ ist, wobei Q die Flussrate der aus dem Katheter austretenden Substanz und r der Außenradius des Katheters ist. Diese Rückfluss- oder Backflowlänge L begrenzt die Einsatzmöglichkeiten eines Katheters und beschränkt insbesondere die Orte, bei welchen ein Katheter eingesetzt werden kann, wenn eine ausreichende Zufuhr einer zu verabreichenden Substanz sichergestellt werden soll.

Wenn zum Beispiel eine intracraniale Oberfläche, wie zum Beispiel ein Sulcus, von dem für eine Infusion oder Injektion verwendeten Katheter durchstoßen wird, wird sich das rückfließende Fluid sehr wahrscheinlich entlang der Oberfläche des Sulcus verteilen und wird nicht das gewünschte Ziel vor der Katherspitze erreichen.

Eine aus einer Öffnung des Katheters austretende Substanz wird erst dann in den Zellenzwischenraum des zu behandelnden Gewebes einfließen, wenn der Druck, welchen das an der Außenseite des Katheters anliegende Gewebe auf die aufsteigende Flüssigkeit ausübt, größer ist als der Druck oder Widerstand des Zellzwischenraum des vor der Austrittsöffnung des Katheters liegenden Gewebes. Es treten häufig Rückflusswege von 2 bis 3 cm auf, so dass zum Beispiel ein 1 cm unter einer Einstichstelle liegendes Gewebe gar nicht behandelt werden kann.

Aus der US 2003/0135200 A1 ist ein Katheter bekannt, welcher einen aus einer einzigen Sinuswelle bestehenden wellenförmigen Bereich aufweist, der als Abdichtung zwischen dem Katheter und einer Harnröhre dienen soll und welcher das Einführen des Katheters in eine gewünschte Einführtiefe ermöglichen soll.

Ein Intracranial-Katheter gemäß Oberbegriff von Anspruch 1 ist z.B. aus US 2003/0097116 bekannt.

Es ist eine Aufgabe der vorliegenden Erfindung einen Katheter vorzuschlagen, welcher es ermöglicht, dass eine vorgegebene Menge einer Substanz sicher und zuverlässig einem bestimmten Körper- oder Gewebebereich zugeführt werden kann.

Diese Aufgabe wird durch den Katheter wie im unabhängigen Anspruch definiert gelöst. Vorteilhafte Ausführungsformen ergeben sich aus den abhängigen Ansprüchen.

Ein erfindungsgemäßer Katheter weist ein Außenprofil auf, das so ausgebildet ist, dass die Länge eines Weges in Längsrichtung oder Zuführrichtung des Katheters entlang zumindest eines Teils oder Abschnitts der Außenoberfläche des Katheters, das heißt die Länge eines Rückflussweges einer aus einer vorderen oder seitlichen Öffnung des Katheters austretenden Substanz, größer ist als die korrespondierende Länge des Katheters, also die Länge, mit welcher zum Beispiel in ein Gewebe eingedrungen werden kann. Der erfindungsgemäße Katheter weist folglich ein Außenprofil auf, welches einer entlang des Katheters rückfließenden Substanz zum Beispiel von einem distalen Katheterende zu einem Infusionspunkt, bis zu welchem ein Katheter in einen bestimmten Körperbereich oder in einen Körper eingestochen wird, eine möglichst lange Wegstrecke entlang der Katheteraußenseite bietet, so dass der notwendige Druck des Gewebes auf die Flüssigkeit in einer möglichst kurzen Distanz entfernt von, also zum Beispiel oberhalb der Katheteröffnung aufgebaut werden kann und somit ein möglichst großer Teil der abzugebenden Substanz an einen Bereich vor oder in der Nähe der Katheteröffnung oder Katheröffnungen zugeführt wird. Die Außenoberfläche oder das Außenprofil eines Katheters ist so geformt, dass die Länge einer sich entlang der Katheterlängsachse oder Kathetermittelachse erstreckenden Strecke auf der Katheteraußenfläche größer ist als der zugehörige Abschnitt des Katheters bzw. als die entsprechende Katheterlänge, wobei der Abstand der Katheteraußenfläche von einer Längs-oder Mittellinie des Katheters entlang der Längsrichtung des Katheters vorzugsweise nicht konstant ist und abschnittsweise oder periodisch zum Beispiel wellenförmig variieren kann.

Durch die erfindungsgemäße Vergrößerung des Rückflussweges durch eine Außenoberfläche des Katheters, welche entlang jedes möglichen oder eines Teils der möglichen Rückflusswege eine im Vergleich zu einem etwa schlauch- oder zylinderförmigen Katheter vergrößerte Rückflusslänge aufweist, kann die Substanz- oder Fluidzufuhr zu Gewebe, wie zum Beispiel zu der weißen Gehirnsubstanz, verbessert und präzisiert werden, wodurch es auch möglich ist, die Vorhersagbarkeit der Fluidverteilung im Gewebe zu erhöhen und somit Simulationen genauer durchführen zu können. Je besser die Fluid- oder Substanzverteilung vorhergesagt werden kann, desto zuverlässiger und effizienter kann der Einsatz eines Katheters, wie zum Beispiel dessen Positionierungsort und/oder die Parameter zur Abgabe einer Flüssigkeit, wie zum Beispiel Druck- oder Flussrate, geplant werden, wodurch der Behandlungserfolg bei der Verwendung des Katheters verbessert werden kann.

Vorzugsweise ist der Katheter so ausgebildet, dass sich die Außenoberfläche oder das Profil oder die Form des Katheters abschnittsweise und/oder periodisch in Längsrichtung des Katheters, also von einem proximalen zu einem distalen Ende, oder nur über einen Abschnitt des Katheters verändert. Beispielsweise kann der Katheter wellenförmig ausgebildet sein, wobei der Katheter zum Beispiel einen in etwa konstanten Querschnitt aufweisen kann und wellenförmig entlang einer Längsrichtung des Katheters ausgebildet ist, wie zum Beispiel in den Figuren 1 und 2 gezeigt. Ebenso kann der Katheter so ausgebildet sein, dass sich der Querschnitt oder Außendurchmesser des Katheters entlang der Katheterlänge verändert, wobei der Katheter eine Wellenform mit Abschnitten kleinerer und größerer Außendurchmesser aufweisen kann, wie beispielhaft in Figur 3 gezeigt.

Der Katheter kann eine oder mehrere Öffnungen aufweisen, welche zum Beispiel an dem vorderen oder distalen Ende und/oder der Seite des Katheters vorgesehen sein können.

Es ist auch möglich den Katheter aus einem porösen oder durchlässigen Material mit oder ohne Katheteröffnungen auszubilden, so dass eine in den Katheter eingebrachte Substanz auch durch Diffusion aus dem Katheter austreten kann.

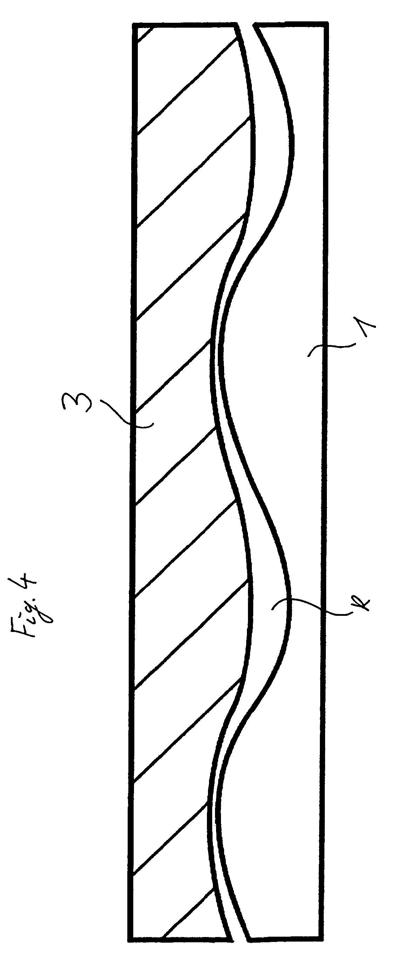

Bei einem Verfahren zur Bestimmung einer Katheterform, insbesondere zur Bestimmung des Außenprofils, wird der Rückflussweg entlang der Katheteraußenseite, also zum Beispiel die Amplitude und/oder die Wellenlänge des Rückflussweges einer Substanz entlang der Oberfläche eines Katheters, welcher sich zum Beispiel bei einem Querschnitt in Längsrichtung des Katheters darstellen lässt, wie in Figur 4 gezeigt, optimiert und zum Beispiel maximiert, wobei zur Bestimmung einer Katheteraußenform und/oder Katheterlänge die Gewebeelastizität so berücksichtigt wird, dass die Kontaktfläche zwischen der Katheteroberfläche und dem durchgedrungenen Gewebe optimiert und möglichst maximiert wird, um keinen oder nur einen kleinen Rückflusskanal für die zu verabreichende Substanz zu bilden. Die Kontaktfläche zwischen der Katheteroberfläche und dem Gewebe bestimmt die Rückflussdistanz, so dass auf Grund der kurvigen oder gekrümmten und nicht gerade oder parallel zur Längsachse verlaufenden Oberfläche die tatsächliche Rückflussdistanz in dem Gewebe verkürzt werden kann. Ebenso können die Konduktivität und/oder Infusionparameter, wie zum Beispiel die Flussrate oder ein Infusionsdruck bei der Auswahl der Katheterform berücksichtigt werden.

Die Erfindung wird nachfolgend anhand bevorzugter Ausführungsformen beschrieben. Es zeigen:

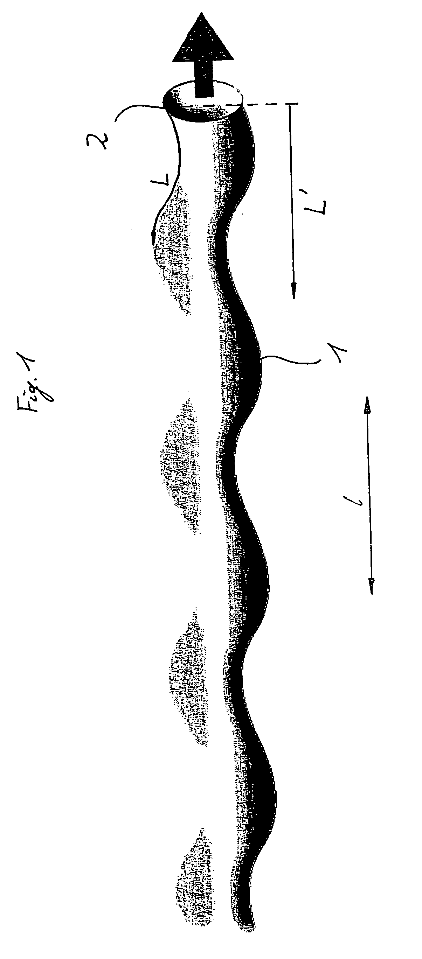

- Figur 1: eine erste Ausführungsform eines wellenförmigen Katheters mit einer distalen Katheteröffnung;
- Figur 1: eine zweite Ausführungsform eines wellenförmigen Katheters mit seitlichen Katheteröffnungen;

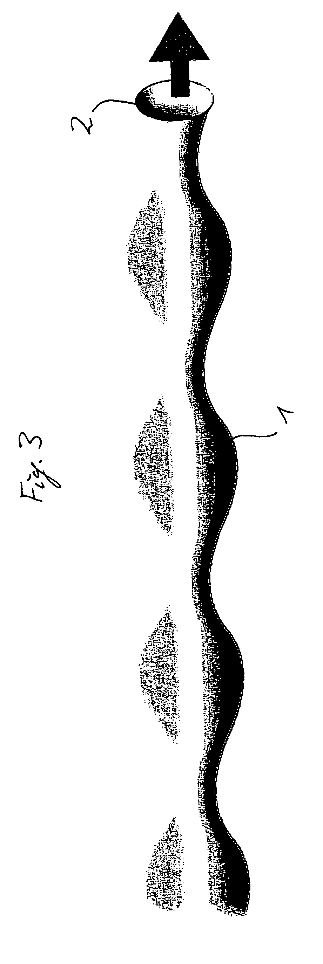

- Figur 3: eine dritte Ausführungsform eines wellenförmigen Katheters mit sich veränderndem Querschnitt; und
- Figur 4: ein Prinzipschaubild zur Erläuterung der Funktionsweise oder der Formbestimmung eines erfindungsgemäßen Katheters.

Figur 1 zeigt einen wellenförmigen Katheter 1 mit einem in Längsrichtung 1 in etwa konstanten Querschnitt, wobei die Kathetermittellinie als Mittelpunkt zwischen gegenüberliegenden Außenpunkten des Katheters 1 in Längsrichtung 1 wellenförmig ausgebildet ist, wodurch die Länge der Außenoberfläche des Katheters 1 in Längsrichtung 1 vergrößert werden kann. Eine aus der distalen Katheteröffnung 2 austretende Substanz, welche entlang der Katheteraußenseite eine Länge L zurückfließt, wird in Längsrichtung 1 des Katheters 1 weniger weit in proximale Richtung fließen, als dies bei einem konventionellen gerade ausgebildeten Katheter mit der auf der Katheteraußenseite gleich langen Rückflusslänge L' der Fall wäre, da der Rückflussweg auf der Katheteraußenoberfläche vergrößert ist.

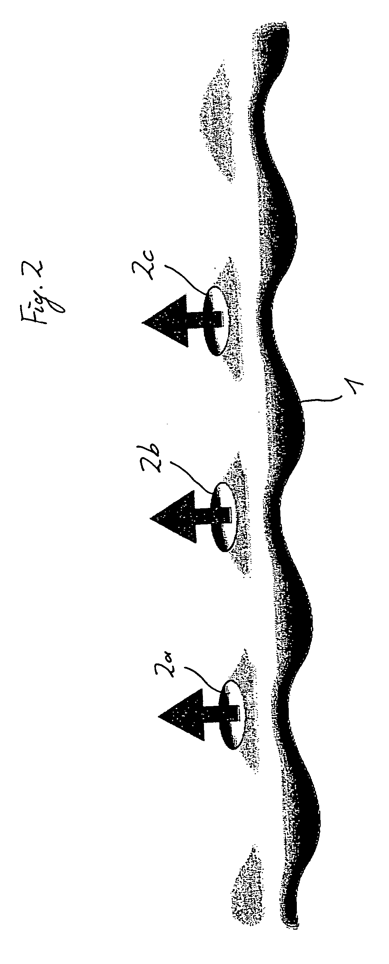

Figur 2 zeigt eine zweite Ausführungsform eines erfindungsgemäßen Katheters 1 mit seitlichen Öffnungen 2a, 2b und 2c zur Abgabe einer durch den Katheter 1 zugeführten Substanz, wobei die aus den Öffnungen 2a bis 2c austretenden Substanz nur einen kleineren Weg entlang der Katheteraußenseite fließt als dies bei bekannten gerade verlaufenden Kathetern der Fall ist.

Figur 3 zeigt eine dritte Ausführungsform eines erfindungsgemäßen Katheters 1 mit distaler Katheteröffnung 2, wobei sich der Querschnitt des Katheters 1 in Längsrichtung des Katheters 1 verändert und wellenförmig ausgebildet ist.

Figur 4 zeigt ein Prinzipschaubild zur Erläuterung der Erfindung, wobei ein Katheter 1 entlang eines Einstichkanals oder einer Trajektorie in das Gewebe 3 eingeführt wurde und mit seiner wellenförmigen Außenseite an dem durchstochenen Gewebe 3 anliegt. Zwischen dem Katheter 1 und dem Gewebe 3 bildet sich ein Rückflusskanal R, wobei das Außenprofil und/oder das Material des Katheters 1 erfindungsgemäß so ausgewählt wird, dass sich unter Berücksichtigung der Elastizität des jeweiligen Gewebes 3 eine möglichst gute Anlage oder eine möglichst große Kontaktfläche der Außenseite des Katheters 1 mit dem Gewebe 3 ergibt, um den Rückflusskanal R zu minimieren. Dabei ist es vorteilhaft die Amplitude der welligen Außenform des Katheters 1 umso größer zu wählen, je größer die Elastizität des Gewebes 3 ist. Vorzugsweise sollte auch die wellenlänge des Außenprofils des Katheters 1 kleiner sein, wenn die Elastizität des Gewebes 3 groß ist.

## Patentansprüche

1. Intracranial-Katheter mit mindestens einer vorderen oder seitlichen Öffnung (2; 2a, 2b, 2c) und mit einem Außenprofil, das so ausgebildet ist, dass die Länge eines Weges entlang zumindest eines Teils der Außenoberfläche des Katheters (1) größer ist als die korrespondierende Länge des Teils des Katheters (1), **dadurch gekennzeichnet, dass** die Außenoberfläche des Katheters (1) sich periodisch in Längsrichtung ändernd oder wellenförmig ausgebildet ist, so dass die Länge eines Rückflussweges entlang der Außenoberfläche des Katheters (1) einer aus einer vorderen oder seitlichen Öffnung des Katheters (1) austretenden Substanz größer ist als die korrespondierende Länge des Katheters (1).

2. Intracranial-Katheter nach einem der vorhergehenden Ansprüche, wobei der Querschnitt des Katheters (1) in Längsrichtung im Wesentlichen konstant bleibt.

3. Intracranial-Vorrichtung nach Anspruch 1, wobei der Querschnitt des Katheters (1) entlang der Längsrichtung des Katheters (1) nicht konstant und bevorzugt sich periodisch ändernd oder wellenförmig ausgebildet ist.

4. Intracranial-Katheter nach einem der vorhergehenden Ansprüche, wobei entlang unterschiedlicher Abschnitte in Längsrichtung des Katheters (1) verschiedene Querschnitte und/oder Katheterformen vorgesehen sind.

5. Intracranial-Katheter nach einem der vorhergehenden Ansprüche mit einer oder mehr Katheteröffnungen (2) am distalen Ende und/oder an der Seite des Katheters (1).

6. Intracranial-Katheter nach einem der vorhergehenden Ansprüche aus einem porösen Material.

## Claims

1. An intracranial catheter having at least one front or lateral opening (2; 2a, 2b, 2c) and an outer profile which is formed such that the length of a path along at least a portion of the outer surface of the catheter (1) is greater than the corresponding length of the portion of the catheter (1), **characterised in that** the outer surface of the catheter (1) is formed undulating or periodically varying in the longitudinal direction, such that the length along the outer surface of the catheter (1) of a reverse flow path of a substance exiting a front or lateral opening of the catheter (1) is greater than the corresponding length of the catheter (1).

2. The intracranial catheter according to the preceding claim, wherein the cross-section of the catheter (1) remains substantially constant in the longitudinal direction.

3. The intracranial catheter according to claim 1, wherein the cross-section of the catheter (1) is not constant along the longitudinal direction of the catheter (1) and is preferably formed periodically varying or undulating.

4. The intracranial catheter according to any one of the preceding claims, wherein various cross-sections and/or shapes of the catheter are provided along different sections in the longitudinal direction of the catheter (1).

5. The intracranial catheter according to any one of the preceding claims, comprising one or more catheter openings (2) at the distal end and/or the side of the catheter (1).

6. The intracranial catheter according to any one of the preceding claims, made of a porous material.

## Revendications

1. Cathéter intracrânien avec au moins un orifice avant ou latéral (2 ; 2a, 2b, 2c) et avec un profil extérieur qui est réalisé de manière que la longueur d'un parcours le long d'au moins une partie de la surface extérieure du cathéter (1) soit supérieure à la longueur correspondante de cette partie du cathéter (1), **caractérisé en ce que** la surface extérieure du cathéter (1) est réalisée de manière à varier périodiquement dans la direction longitudinale ou est réalisée ondulée, ce qui fait que la longueur d'une voie de reflux le long de la surface extérieure du cathéter (1) d'une substance sortant d'un orifice avant ou latéral du cathéter (1), est supérieure à la longueur correspondante du cathéter (1).

2. Cathéter intracrânien selon la revendication précédente, dans lequel la section transversale du cathéter (1) dans la direction longitudinale est sensiblement constante.

3. Dispositif intracrânien selon la revendication 1, dans lequel la section transversale du cathéter (1) le long de la direction longitudinale du cathéter (1) n'est pas constante et est réalisée de préférence de manière à varier périodiquement ou est réalisée ondulée.

4. Cathéter intracrânien selon l'une quelconque des revendications 1 à 3, dans lequel il est prévu différentes sections transversales et/ou formes de cathéter le long de différents segments dans la direction longitudinale du cathéter (1).

5. Cathéter intracrânien selon l'une quelconque des revendications 1 à 4 avec un ou plusieurs orifices de cathéter (2) à l'extrémité distale et/ou sur le côté du cathéter (1).

6. Cathéter intracrânien selon l'une quelconque des revendications 1 à 5 constitué d'un matériau poreux.
